# FASCICULE DE BREVET EUROPEEN

(11) **EP 0 549 774 B1**
(45) Date de publication et mention de la délivrance du brevet: **27.09.2000**
(21) Numéro de dépôt: 92916083.6
(22) Date de dépôt: 20.07.1992
(51) Int. Cl.: C12N 5/00

(54) **UTILISATION DE CARBONATE DE CALCIUM POREUX COMME MATERIAU SUPPORT DE CULTURE DE CELLULES IN VITRO**
Verwendung von porösem Calciumcarbonat als Trägermaterial für in vitro Zellkulturen.
USE OF POROUS CALCIUM CARBONATE AS A SUPPORT MATERIAL FOR IN VITRO CELL CULTURE

(30) Priorité: 19.07.1991 FR 9109205
(43) Date de publication de la demande: 07.07.1993
(73) Titulaire: BIO HOLDINGS INTERNATIONAL LIMITED, Tortola (VG)
(72) Inventeur: GUILLEMIN, Geneviève, F-75017 Paris (FR); CHRISTEL, Pascal, F-75018 Paris (FR); PATAT, Jean-Louis, F-75010 Paris (FR); MEUNIER, Alain, F-77120 Coulommiers (FR)
(74) Mandataire: Hubert, Philippe
(86) Numéro de dépôt international: FR9200707
(87) Numéro de publication internationale: WO9302181

(56) Documents cités:
- EP-A- 0 175 286
- FR-A- 2 223 325
- US-A- 3 890 107
- CHEMICAL ABSTRACTS, vol. 111, no. 6, 7 Août 1989, Columbus, Ohio, US; abstract no. 45236h, G. GUILLEMIN ET AL. 'COMPARISON OF CORAL RESORPTION AND BONE APPOSITION WITH TWO NATURAL CORALS OF DIFFERENT POROSITIES.' page 383 ;
- CHEMICAL ABSTRACTS, vol. 107, no. 7, 17 Août 1987, Columbus, Ohio, US; abstract no. 56902r, G. GUILLEMIN ET AL. 'THE USE OF CORAL AS A BONE GRAFT SUBSTITUTE.' page 553 ;

## Description

La présente invention a pour objet l'utilisation de carbonate de calcium poreux, sous forme d'aragonite, et en particulier l'utilisation de squelette de corail poreux comme matériau support de culture in vitro de cellules eucaryotes ou procaryotes.

On sait que la plupart des cellules eucaryotes ne peuvent être cultivées in vitro que sur un support solide. De telles cultures sont généralement effectuées dans des conteneurs ou fioles dans lesquelles la multiplication cellulaire s'arrête lorsqu'une couche monocellulaire tapisse entièrement la partie de la paroi en contact avec le milieu nutritif liquide.

De tels procédés de culture présentent l'inconvénient de nécessiter la manipulation d'un nombre élevé de conteneurs.

Par ailleurs, les cellules eucaryotes ne croissent pas sur certains matériaux, et il n'est pas possible de prédire si un matériau donné conviendra à la culture in vitro de ces cellules.

Il existe actuellement des besoins importants de culture in vitro de cellules eucaryotes dans divers domaines.

Par exemple, la culture in vitro de cellules végétales est susceptible de permettre la production et l'isolement de substances naturelles en s'affranchissant des limitations et des aléas de la culture en pleine terre.

Les cellules végétales sont nettement plus sensibles que les bactéries aux effets de cisaillement provoqués par les systèmes habituellement employés dans les fermenteurs. Il est donc intéressant de cultiver ces cellules sur support solide. Sous l'action d'hormones, dont la concentration est définie dans chaque cas, on peut cultiver à l'état isolé et en conditions stériles n'importe quel fragment d'une plante. Les cellules indifférentiées (cals) sont capables de synthétiser des métabolites qui normalement ne se retrouvent que dans certains organes spécifiques de la plante.

A l'heure actuelle, ce sont surtout des principes actifs médicamenteux qui sont produits, de même que diverses essences, arômes, colorants et pesticides dont la complexité moléculaire rend la synthèse chimique trop difficile et/ou trop onéreuse.

A titre d'applications des cultures de cellules végétales, on peut citer :
- production d'anthraquinones par GALLIUM APARINE ;
- Production d'un anticancéreux, la tripdiolide, par TRYTERIZHIUM WILFORDII ;
- Production d'un antispasmodique (l'atropine) par ATROPE BELLADONA ;
- production d'alcaloïdes par PAPAVER SOMNIFERUM.

De plus, les cellules végétales maintenues in vitro ont souvent de fortes capacités de biotransformation. Par exemple un dérivé de l'acide salicylique présente une activité analgique plus rapide que celle de l'aspirine ainsi qu'une meilleure tolérance gastrique.

De même, il est souhaitable de pouvoir cultiver in vitro de nombreuses cellules animales, par exemple dans un but médical. On sait que les cellules de moelle osseuse, qui sont des précurseurs des cellules du système immunitaire, sont très difficiles à cultiver in vitro, bien qu'une telle culture présente un grand intérêt dans le domaine des allogreffes ou des autogreffes.

L'intérêt d'une bonne multiplication cellulaire in vitro s'étend aux cellules modifiées par recombinaison génétique. Jusqu'à présent de telles cellules ne peuvent être cultivées, en pratique, que si elles sont immortalisées par hybridation avec des cellules cancéreuses ou par transformation à l'aide d'un virus.

Un autre intérêt de la culture de cellules in vitro serait, chez un patient souffrant d'une perte de substance osseuse, actuelle ou prévisible, le prélèvement de cellules osseuses chez ce patient, la mise en culture in vitro de ces cellules sur un support convenable, et ultérieurement l'introduction dudit support comprenant les cellules ainsi cultivées afin de combler la perte de substance. On sait en effet que le corail peut constituer une prothèse osseuse biodégradable, dont la réhabitation par l'os à mesure de la dégradation sera favorisée par des cellules cultivées ainsi réintroduites.

Il est également intéressant d'améliorer les procédés de culture de lignées cellulaires telles que les lignées CHO, VERO, ou les hybridomes, en vue de la production de protéines, de vaccins, d'hormones, d'anticorps, etc ...

Il en va de même pour les cultures de cellules d'insectes en vue de la production de protéines recombinantes ou de virus (par exemple baculovirus).

Un autre domaine intéressant est la culture de champignons filamenteux, notamment d'ascomycètes, en particulier de levures (par exemple Aspergillus, penicillium, etc ...). On peut citer plus particulièrement la culture d'A. flavius pour la production d'aflatoxines, ou la culture d'A. niger pour la production de différents acides organiques.

On a maintenant découvert que le carbonate de calcium poreux, sous la forme d'aragonite, et notamment le squelette de corail poreux, constitue un matériau support particulièrement intéressant pour la culture in vitro de cellules, et en particulier des divers types de cellules mentionnés ci-dessus.

Dans la suite de la description on fera référence par commodité au squelette de corail, dont on sait qu'il est constitué de cristaux de carbonate de calcium (aragonite), ou plus simplement au corail, mais il convient de préciser que ces expressions doivent être interprétées ici comme désignant tout matériau calcaire poreux, naturel ou synthétique, àbase d'aragonite notamment sous forme polycristalline.

Le document EP-A-0175286 décrit la culture de cellules de mammifères sur des supports solides à base de dérivés du calcium, y compris à base de carbonate de calcium sous forme de calcite.

La présente invention a donc pour objet l'utilisation d'un matériau poreux, constitué essentiellement d'aragonite, comme support solide tridimensionnel de culture in vitro de cellules eucaryotes.

Pour que le corail puisse être utilisé comme véritable support de culture tridimensionnel, il convient de l'utiliser sous la forme de fragments ayant généralement des dimensions supérieures à 0,5 mm et en particulier supérieures à 1 mm.

Ce matériau peut se présenter par exemple sous la forme de cylindres, de sphères, de plaques, etc...

De tels matériaux peuvent être obtenus par exemple de la façon suivante : les morceaux de corail brut sont abondamment rincés à l'eau courante, séchés et débités en tronçons, puis mis sous la forme et aux dimensions désirées, selon les méthodes connues. Ces tronçons sont plongés dans une solution d'hypochlorite de sodium à 12 g/l pendant 48 heures, puis rincés à l'eau courante pendant 48 heures, et séchés. Finalement, on effectue une stérilisation par rayonnement gamma.

L'un des intérêts de l'utilisation du squelette de corail est que, dans ce squelette, les pores sont communiquants, de sorte que toutes les parties du squelette, même sous forme de pièces de dimensions relativement importantes, sont utilisables. Cette communication des pores favorise en outre un envahissement complet du matériau support par les cellules cultivées, et le rendement de la culture, rapporté au volume de matériau support utilisé, est donc optimum. Ce rendement volumique est évidemment élevé, puisque le corail constitue un support tridimensionnel.

De préférence, on utilise comme matériau support un matériau ayant des diamètres de pores compris entre 50 et 250 µm, avec une porosité, c'est-à-dire le volume des pores par rapport au volume total du matériau, comprise généralement entre 20 et 80 %.

C'est le cas notamment avec le corail des genres Porites, Acropora, Goniopora, Lobophyllia, Symphyllia, et Millipora.

Bien entendu, les milieux et conditions de culture utilisés selon l'invention sont ceux qui conviennent à la cellule particulière qui est cultivée. Ces milieux et conditions de cultures sont bien connus ou peuvent être déterminés par de simples expériences de routine.

L'invention concerne l'utilisation de squelette de corail comme matériau support dans la culture in vitro de toutes cellules eucaryotes, notamment végétales ou animales, y compris des cellules humaines.

Il peut s'agir par exemple de fibroblastes, de cellules endothéliales, de cellules de moelle osseuse, y compris les cellules souches totipotentes, les ostéoblastes ; ou encore des cellules transformées telles que des hybridomes, etc...

L'invention a également pour objet un procédé pour cultiver des cellules eucaryotes in vitro, caractérisé par le fait que l'on ensemence avec lesdites cellules un milieu de culture comprenant un support solide tridimensionnel poreux en aragonite, immergé dans un milieu nutritif liquide approprié.

Le matériau support, le milieu de culture et/ou lesdites cellules eucaryotes sont en particulier tels que définis précédemment.

La culture est effectuée à un pH convenant à la multiplication de la cellule cultivée. Ce pH est de préférence supérieur à 7, pour éviter une dissolution notable du matériau support. Si nécessaire, on ajuste le pH en cours de culture à l'aide d'un modificateur de pH approprié.

Pour la culture de cellules végétales, on utilise un liquide nutritif contenant notamment des éléments minéraux, une source d'azote et divers additifs comme par exemple les micro-éléments de HELLER.

De façon préférentielle, on utilise pour la culture de cellules végétales des sources de carbone, d'azote, des éléments minéraux et vitaminiques et des hormones spécifiques pour éviter les effets de cisaillement provoqués par les systèmes habituellement employés dans les fermenteurs.

Pour la culture de champignons filamenteux (ascomycètes), le milieu nutritif contient des sources de carbone et d'azote et des éléments minéraux et vitaminiques.

Généralement, la proportion, en volume, du support solide par rapport au volume total (liquide + solide), est supérieure à 5 %, et en particulier supérieure à 10 % ; elle est le plus souvent inférieure ou égale à 50 %.

Pour recueillir les cellules après culture, si désiré, on peut effectuer par exemple une trypsinisation, de façon connue. Dans un mode de réalisation particulier, on peut mettre en oeuvre le procédé de l'invention en cultivant successivement, sur un même support, deux cellules différentes d'une même espèce animale.

On obtient ainsi un support revêtu d'une première couche de cellules elle - même revêtue d'une seconde couche de cellules différentes des cellules de la première couche.

Un tel procédé est intéressant notamment pour cultiver certaines cellules telles que les cellules de moelle osseuse (cellules souches ou ostéoblastes) qui croissent plus facilement sur une assise de cellules (telles que des cellules endothéliales ou des fibroblastes) que directement sur le support solide.

L'invention a également pour objet le produit de culture cellulaire constitué par un support tridimensionnel poreux d'aragonite habité par des cellules eucaryotes, ce produit de culture pouvant notamment être obtenu selon les procédés qui viennent d'être définis, comprenant la culture de deux cellules différentes d'une même espéce animale.

Le produit de culture selon l'invention peut être constitué notamment par le support solide revêtu d'un tapissage de cellules endothéliales ou de fibroblastes. Le produit de culture de l'invention peut également être un support revêtu d'un tel tapissage lui-même revêtu de cellules souches de moelle osseuse et/ou d'ostéoblastes.

Les exemples suivants illustrent l'invention sans toutefois la limiter.

### EXEMPLE 1 : Culture de cellules de moelle osseuse

Il est réalisé un revêtement cellulaire (fibroblastes) d'implants coralliens puis on imprègne ces implants, revêtus de fibroblastes, avec de la moelle osseuse du patient.

### 1. REVETEMENT FIBROBLASTIQUE

### 1.1. Matériel

Matériau : corail Porites (volume de porosité : 50 %) sous forme de pastilles diamètre : 30 mm, épaisseur : 1 mm.

Milieu de culture : M199 supplémenté par 10 % de sérum de veau foetal et 1 % d'antibiotiques (pénicilline-streptomycine).

Type cellulaire : fibroblastes épidermiques de rat.

### 1.2. Méthodes

Obtention d'une culture de fibroblastes épidermiques de rat par la méthode des explants ;
Après avoir été rasée, la peau est pincée à l'aide d'une pince courbe, puis avec un scapel on découpe la peau comprise entre les deux mors de la pince et on dépose le lambeau dans une solution saline contenant des antibiotiques à forte concentration (6 %). Chaque morceau est ensuite redécoupé en petits carrés aux bords nettement tranchés de 2 mm² environ.

On rince les biopsies à trois reprises dans le milieu de culture contenant une concentration de pénicilline streptomycine 6, 4 puis 2 fois supérieure à la concentration normale.

On découpe les biopsies de morceaux de 2 mm² par coupures "franches" ; on les dépose dans des boîtes de culture plastique.

On laisse les biopsies adhérer au support avant d'ajouter le milieu de culture, auquel on aura ajouté du sérum de veau foetal à raison de 10 % minimum. Une bonne adhésion est obtenue en laissant les flacons debout sans milieu, pendant 40 à 60 minutes dans l'incubateur avant de les reposer.

Il convient ensuite de conserver les boîtes immobiles, et au bout de deux ou trois jours, les fibroblastes commencent à proliférer autour des explants. Le milieu doit alors être changé tous les deux ou trois jours car les fibroblastes sont éliminés dès que la prolifération a démarré et la première sous-culture peut être réalisée. Si les fibroblastes coexistent dans des cultures primaires avec des cellules plus délicates à cultiver (comme des myoblastes ou des cellules endothéliales), on sélectionne les fibroblastes par la pauvreté du milieu de culture (à laquelle des fibroblastes résistent mais pas les autres types cellulaires). Lorsque les fibroblastes ont atteint la confluence, c'est-à-dire que l'inhibition de contact les empêches de proliférer davantage, on passe, après repiquage, à une culture tridimensionnelle. Dans ce type de culture, les cellules prolifèrent dans les trois dimensions sur un support tridimensionnel.

Pour réaliser cette culture, ces solutions cellulaires sont déposée sur des pastilles de corail Porites de 1 mm de diamètre et 1 mm d'épaisseur, le tout dans des boîtes 6 puits ou dans des boîtes de pétri de 35 mm de diamètre. Des quantifications de croissance cellulaire sont faites à intervalles réguliers de façon à évaluer l'augmentation du nombre de cellules contenues dans les pastilles coraliennes.

Une fois que le corail est tapissé de fibroblastes, on passe à la deuxième étape : l'imprégnation par des cellules contenues dans de la moelle osseuse fraîche, soit totale, soit épurée des cellules non-nucléées (hématies et adipocytes).

### 2. CULTURE DE CELLULES DE MOELLE OSSEUSE

La moelle osseuse est récupérée par prélèvement chez le rat dans des flacons contenant de la calciparine (3ml/500 ml). Les cellules sont bien dispersées puis mises sur Ficoll à raison de 12 ml de Ficoll pour 20 ml de solution de moelle, à 18°C. L'ensemble est centrifugé à 1 600 tours/min pendant 20 min, dans une centrifugeuse stable ne comportant pas de frein. On peut ainsi isoler les cellules souches.

On ensemence alors le milieu de culture, contenant le corail tapissé de fibroblastes, avec 10⁷ cellules souches par ml de milieu nutritif liquide.

On observe un développement tridimensionnel important des cellules de moelle osseuse.

On a obtenu des résultats analogues en cultivant d'abord des fibroblastes de derme humain, puis des cellules de moelle osseuse humaine.

### EXEMPLE 2 : Culture de fibroblaste embryonnaire de poumon humain.

Le matériau support est constitué de sphères ou de cylindres de squelette de corail du genre Porites. Les sphères ont 3 à 4 mm de diamètre, les cylindres ont des dimensions de 3 mm de diamètre et 4 mm de longueur.

On a utilisé soit du squelette de corail ayant 20 % de porosité, soit du squelette ayant 50 % de porosité. La cellule cultivée est la lignée MRC5, qui est une lignée de fibroblaste embryonnaire de poumon humain.

La culture est mise en oeuvre dans des plaques de culture multipuits. L'inoculation est effectuée à raison de 1 ml de milieu contenant 50 000 cellules.

Le milieu de culture est le milieu Williams supplémenté avec 5 % de sérum de veau nouveau-né. Les supports sont ensuite recouverts avec le milieu de culture. Les plaques sont mises dans une enceinte thermostatée à 37°C sous atmosphère humide (air à 5 % de CO₂). Après 24 heures d'incubation, les supports sont repris stérilement et mis en incubation dans d'autres plaques contenant du milieu neuf à 37°C, et on remet en culture dans les mêmes conditions que précédemment. Ensuite le milieu est changé tous les deux jours, la durée totale de la culture étant de 7 jours.

Au bout de 7 jours de culture, on a évalué la biomasse cellulaire par dosage des protéines selon la méthode de Lowry, ainsi que par la coloration de l'ADN par le bleu de méthylène.

On a observé une bonne croissance cellulaire aussi bien avec les supports sous forme de sphères que sous forme de cylindres.

La croissance est plus forte sur les matériaux à 20 % de porosité que sur les matériaux à 50 % de porosité.

### EXEMPLE 3 : Culture d'un hybridome producteur d'un anticorps monoclonal.

L'hybridome sécrète un anticorps morsoclonal anti-folate.

Il est cultivé en milieu RPMI, supplémenté avec 5 % de sérum de veau nouveau-né.

Les autres conditions de culture sont les mêmes que celles décrites à l'exemple 2.

La croissance cellulaire a été mise en évidence par la détermination, selon la technique ELISA, de la quantité d'anticorps monoclonaux produits.

## Revendications

1. Utilisation d'un matériau poreux polycristalline, constitué essentiellement d'aragonite comme support solide tridimensionnel de culture in vitro de cellules.

2. Utilisation selon la revendication 1, caractérisée par le fait que ledit support est constitué par des fragments de squelette de corail poreux.

3. Utilisation selon l'une quelconque des revendications précédentes, caractérisée par le fait que ledit support se présente sous la forme de cylindres, de sphères ou de plaques.

4. Utilisation selon l'une quelconque des revendications précédentes, caractérisée par le fait que les diamètres des pores sont compris entre 50 et 250 µm.

5. Utilisation selon l'une quelconque des revendications précédentes, caractérisée par le fait que le volume des pores représente de 20 à 80 % du volume total du support.

6. Utilisation selon l'une quelconque des revendications 2 à 5, caractérisée par le fait que ledit corail est choisi parmi les genres Porites, Acropora, Goniopora, Lobophyllia, Symphyllia et Millipora.

7. Utilisation selon l'une quelconque des revendications précédentes, caractérisée par le fait que ledit support solide est immergé dans un milieu nutritif liquide.

8. Utilisation selon la revendication précédente, caractérisée par le fait que la proportion en volume du support solide par rapport au volume total (liquide + solide) est supérieure à 5 % et en particulier supérieure à 10 %.

9. Procédé pour cultiver des cellules eucaryotes in vitro, caractérisé par le fait que l'on ensemence avec lesdites cellules un milieu de culture comprenant un support solide tridimensionnel poreux immergé dans un milieu nutritif liquide approprié, ledit support étant constitué essentiellement d'aragonite polycristalline.

10. Procédé selon la revendication précédente, caractérisé par le fait que ledit support et/ou ledit milieu liquide est tel que défini dans l'une quelconque des revendications 2 à 8.

11. Procédé selon l'une quelconque des revendications 9 et 10, caractérisé par le fait que lesdites cellules sont choisies parmi les cellules végétales, les cellules animales, et les cellules de champignons filamenteux.

12. Procédé selon la revendication précédente, caractérisé par le fait que lesdites cellules sont des cellules humaines, des cellules d'insectes, des cellules végétales indifférenciées, ou des cellules de levures.

13. Procédé selon l'une quelconque des revendications 11 et 12, caractérisé par le fait que lesdites cellules sort choisies parmi les fibroblastes, les cellules endothéliales, les cellules de moelle osseuse, y compris les cellules souches totipotentes, et les ostéoblastes.

14. Procédé selon l'une quelconque des revendications 9 et 10, caractérisé par le fait que lesdites cellules sont des cellules transformées, en particulier des hybridomes.

15. Procédé selon l'une quelconque des revendications 9 à 13, caractérisé par le fait que l'on cultive successivement, sur un même support, deux cellules différentes d'une même espèce animale, de façon à obtenir un support revêtue d'une première couche de cellules elle - même revêtue d'une seconde couche de cellules différentes des cellules de la première couche.

16. Procédé selon la revendication précédente, caractérisé par le fait que l'on cultive d'abord des fibroblastes, ou des cellules endothéliales, puis que l'on cultive ensuite des cellules de moelle osseuse.

17. Procédé selon la revendication précédente, caractérisé par le fait que lesdites cellules de moelle osseuse sont des cellules souches totipotentes ou des ostéoblastes.

18. Produit de culture cellulaire constitué par un support de carbonate de calcium
tridimensionnel poreux habité par des cellules eucaryotes,
caractérisé par le fait qu'il peut être obtenu selon le procédé de l'une quelconque des revendications 15 à 17.

19. Produit selon la revendication 18, caractérisé par le fait que ledit support est revêtu d'un tapissage de cellules endothéliales ou de fibroblastes, et par
le fait que ledit tapissage est lui-même revêtu de cellules souches de moelle osseuse et/ou d'ostéoblastes.

20. Produit selon la revendication 19, caractérisé par le fait que lesdites cellules sont des cellules humaines.

## Claims

1. Use of a polycrystalline porous material, consisting essentially of aragonite, as a three-dimensional solid support for in vitro cell culture.

2. Use according to Claim 1, characterised in that said support consists of fragments of porous coral skeleton.

3. Use according to any one of the preceding claims, characterised in that said support is in the form of cylinders, spheres or plates.

4. Use according to any one of the preceding claims, characterised in that the pore diameters are between 50 and 250 µm.

5. Use according to any one of the preceding claims, characterised in that the pore volume represents from 20 to 80 % of the total volume of the support.

6. Use according to any one of Claims 2 to 5 characterised in that said coral is chosen from the genera Porites, Acropora, Goniopora, Lobophyllia, Symphyllia and Millipora.

7. Use according to any one of the preceding claims, characterised in that said solid support is immersed in a liquid nutrient medium.

8. Use according to the preceding claim, characterised in that the proportion by volume of the solid support relative to the total volume (liquid + solid) is greater than 5 %, and especially greater than 10 %.

9. Method for cultivating eukaryotic cells in vitro, characterised in that a culture medium comprising a porous three-dimensional solid support immersed in a suitable liquid nutrient medium is inoculated with said cells, said support consisting essentially of polycrystalline aragonite.

10. Method according to the preceding claim, characterised in that said support and/or said liquid medium is as defined in any one of Claims 2 to 8.

11. Method according to either of Claims 9 and 10, characterised in that said cells are chosen from plant cells, animal cells and cells of filamentous fungi.

12. Method according to the preceding claim, characterised in that said cells are human cells, insect cells, undifferentiated plant cells or yeast cells.

13. Method according to either of Claims 11 and 12, characterised in that said cells are chosen from fibroblasts, endothelial cells, bone marrow cells including totipotent stem cells and osteoblasts.

14. Method according to either of Claims 9 and 10, characterised in that said cells are transformed cells, especially hybridomas.

15. Method according to any one of Claims 9 to 13, characterised in that two different cells of the same animal species are cultured successively on the same support in order to obtain a support coated with a first layer of cells being itself coated with a second layer of cells different from those of the first layer.

16. Method according to the preceding claim, characterised in that fibroblasts or endothelial cells are cultured first and then bone marrow cells are cultured thereafter.

17. Method according to the preceding claim, characterised in that said bone marrow cells are totipotent strain cells or osteoblasts.

18. Cell culture product, consisting of a three-dimensional, porous calcium carbonate support occupied by eukaryotic cells, which can be obtained according to the method of any one of claims 15 to 17.

19. Product according to Claim 18, characterised in that said support is coated with a layer of endothelial cells or fibroblasts, and characterised in that said layer is itself coated with bone marrow stem cells and/or osteoblasts.

20. Product according to Claim 19, characterised in that said cells are human cells.

## Patentansprüche

1. Verwendung eines im Wesentlichen aus Aragonit bestehenden porösen polykristallinen Materials als dreidimensionaler fester Träger für eine In-vitro-Zellkultur.

2. Verwendung nach Anspruch 1, **dadurch gekennzeichnet, dass** der Träger aus porösen Korallenskelettfragmenten besteht.

3. Verwendung nach einem der vorhergehenden Ansprüche, **dadurch gekennzeichnet, dass** der Träger in Form von Zylindern, Kugeln oder Platten vorliegt.

4. Verwendung nach einem der vorhergehenden Ansprüche, **dadurch gekennzeichnet, dass** der Porendurchmesser 50 bis 250 µm beträgt.

5. Verwendung nach einem der vorhergehenden Ansprüche, **dadurch gekennzeichnet, dass** das Porenvolumen 20 bis 80 % des Gesamtvolumens des Trägers ausmacht.

6. Verwendung nach einem der Ansprüche 2 bis 5, **dadurch gekennzeichnet, dass** die Koralle aus den Gattungen Porites, Acropora, Goniopora, Lobophyllia, Symphyllia und Millipora ausgewählt ist.

7. Verwendung nach einem der vorhergehenden Ansprüche, **dadurch gekennzeichnet, dass** der feste Träger in ein flüssiges Nährmedium eingetaucht ist.

8. Verwendung nach dem vorhergehenden Anspruch, **dadurch gekennzeichnet, dass** der Volumenanteil des festen Trägers am Gesamtvolumen (flüssig + fest) mehr als 5 % und insbesondere mehr als 10 % beträgt.

9. Verfahren zur Kultivierung von Eukaryontenzellen in vitro, **dadurch gekennzeichnet, dass** mit diesen Zellen ein Kulturmedium beimpft wird, das einen dreidimensionalen porösen festen Träger umfasst, der in ein geeignetes flüssiges Nährmedium eingetaucht ist, wobei der Träger im Wesentlichen aus polykristallinem Aragonit besteht.

10. Verfahren nach dem vorhergehenden Anspruch, **dadurch gekennzeichnet, dass** der Träger und/oder das flüssige Medium wie in einem der Ansprüche 2 bis 8 definiert ist/sind.

11. Verfahren nach Anspruch 9 oder 10, **dadurch gekennzeichnet**, **dass** die Zellen aus Pflanzenzellen, tierischen Zellen und Fadenpilzzellen ausgewählt sind.

12. Verfahren nach dem vorhergehenden Anspruch, **dadurch gekennzeichnet, dass** die Zellen menschliche Zellen, Insektenzellen, undifferenzierte Pflanzenzellen oder Hefezellen sind.

13. Verfahren nach Anspruch 11 oder 12, **dadurch gekennzeichnet**, **dass** die Zellen aus Fibroblasten, Endothelzellen, Knochenmarkzellen, eingeschlossen die totipotenten Stammzellen, und Osteoblasten ausgewählt sind.

14. Verfahren nach Anspruch 9 oder 10, **dadurch gekennzeichnet**, **dass** die Zellen umgewandelte Zellen, insbesondere Hybridzellen, sind.

15. Verfahren nach einem der Ansprüche 9 bis 13, **dadurch gekennzeichnet, dass** auf ein und demselben Träger nacheinander zwei verschiedene Zellen ein und derselben Tierspezies derart kultiviert werden, dass ein Träger erhalten wird, der mit einer ersten Zellschicht überzogen ist, die ihrerseits mit einer zweiten Zellschicht überzogen ist, deren Zellen sich von denen der ersten Schicht unterscheiden.

16. Verfahren nach dem vorhergehenden Anspruch, **dadurch gekennzeichnet, dass** zuerst Fibroblasten oder Endothelzellen und anschließend Knochenmarkzellen kultiviert werden.

17. Verfahren nach dem vorhergehenden Anspruch, **dadurch gekennzeichnet, dass** die Knochenmarkzellen totipotente Stammzellen oder Osteoblasten sind.

18. Erzeugnis aus einer Zellkultur, das aus einem dreidimensionalen porösen Calciumcarbonatträger besteht, der von Eukaryontenzellen besiedelt ist, **dadurch gekennzeichnet**, **dass** es gemäß dem Verfahren nach einem der Ansprüche 15 bis 17 erhalten werden kann.

19. Erzeugnis nach Anspruch 18, **dadurch gekennzeichnet, dass** der Träger mit einer Schicht aus Endothelzellen oder Fibroblasten überzogen ist **und dass** diese Schicht ihrerseits mit Knochenmarkstammzellen und/oder Osteoblasten überzogen ist.

20. Erzeugnis nach Anspruch 19, **dadurch gekennzeichnet, dass** die Zellen menschliche Zellen sind.
